# EUROPEAN PATENT APPLICATION

(11) **EP 0 630 974 A2**
(43) Date of publication of application: **28.12.1994**
(21) Application number: 94201768.2
(22) Date of filing: 21.06.1994
(51) Int. Cl.: G01N 33/84, C12Q 1/68

(54) **Method and test kit for the detection of inorganic orthophosphate by-product from amplification of target nucleic acid**

(30) Priority: 25.06.1993 US 81507
(71) Applicant: Johnson & Johnson Clinical Diagnostics, Inc., Rochester New York 14650 (US)
(72) Inventor: Friedman, Alan E., c/o Eastman Kodak Company, Rochester, New York 14650-2201 (US); Cummins, Thomas J., c/o Eastman Kodak Company, Rochester, New York 14650-2201 (US)
(74) Representative: Mercer, Christopher Paul

(57) **Abstract**

The presence of amplified nucleic acids can be identified by detecting the presence of inorganic orthophosphate which is a stable by-product of the amplification process. There are a number of useful detection systems which can be used in either solution or dry formats. A preferred embodiment is to use a dry analytical element containing suitable reagents for generating a colorimetric signal in response to the orthophosphate. A diagnostic test kit can include individually packaged reagents for both amplification and orthophosphate detection.

SEQUENCE LISTING

(1) GENERAL INFORMATION
(i) APPLICANT
(A) NAME: Eastman Kodak Company
(B) STREET: 343 State Street
(C) CITY: Rochester
(D) STATE: New York
(E) COUNTRY: USA
(F) POSTAL CODE: 14650-2201
(ii) TITLE OF THE INVENTION: Method and Test Kit
for the Detection of Inorganic Orthophosphate By-
Product from Amplification of Target Nucleic Acid
(iii) NUMBER OF SEQUENCES: 2
(iv) COMPUTER READABLE FORM:
(A) MEDIUM TYPE: Diskette, 3.5 inch,
1.44 MB storage (IBM)
(B) COMPUTER: IBM PS/2
(C) OPERATING SYSTEM: MS-DOS Version 3.3
(D) SOFTWARE: PC-8 (Word for Windows)

(2) INFORMATION FOR SEQ ID NO:1:
(i) SEQUENCE CHARACTERISTICS:
(A) LENGTH: 28 nucleotides
(B) TYPE: Nucleic acid
(C) STRANDEDNESS: Single
(D) TOPOLOGY: Linear
(ii) MOLECULE TYPE: Primer for HIV-I DNA
(iii) HYPOTHETICAL: No
(iv) ANTI-SENSE: No
(vi) ORIGINAL SOURCE: Synthetically prepared
(vii) IMMEDIATE SOURCE: Same
(x) PUBLICATION INFORMATION: None
(xi) SEQUENCE DESCRIPTION: SEQ ID NO:1:

AGTGGGGGGA CATCAAGCAG CCATGCAA 28

(3) INFORMATION FOR SEQ ID NO:2:
(i) SEQUENCE CHARACTERISTICS:
(A) LENGTH: 28 nucleotides
(B) TYPE: Nucleic acid
(C) STRANDEDNESS: Single
(D) TOPOLOGY: Linear
(ii) MOLECULE TYPE: Primer for HIV-I DNA
(iii) HYPOTHETICAL: No
(iv) ANTI-SENSE: No
(vi) ORIGINAL SOURCE: Synthetically prepared
(vii) IMMEDIATE SOURCE: Same
(x) PUBLICATION INFORMATION: None
(xi) SEQUENCE DESCRIPTION: SEQ ID NO:2:

TTCCTGCTAT GTCACTTCCC CTTGGTTC 28

## Description

This invention relates to diagnostic methods for the detection of nucleic acids by monitoring the production of a by-product associated with amplification procedures. In particular, it relates to a test kit and a method to detect a target nucleic acid by measuring the inorganic orthophosphate generated when the target nucleic acid is amplified using various amplification procedures, including polymerase chain reaction (PCR).

Technology to detect minute quantities of nucleic acids has advanced rapidly over the last two decades including the development of highly sophisticated hybridization assays using probes in amplification techniques such as PCR, ligase chain reaction and self-sustained sequence replication. Researchers have readily recognized the value of such technologies to detect diseases and genetic features in human or animal test specimens.

In particular, PCR is a significant advance in the art to allow detection of very small concentrations of a target nucleic acid. The details of PCR are described, for example, in US-A-4,683,195; US-A-4,683,202; and US-A-4,965,188; although there is a rapidly expanding volume of literature in this field.

Despite the broad and rapid use of PCR in a variety of biological and diagnostic fields, there are still practical limitations which must be overcome to achieve the optimum success of the technology.

PCR requires considerable expensive reagents, especially in various detection systems. Detection of the amplified target nucleic acid has generally required the use of ethidium bromide gels or expensive capture and detection probes. The use of such materials requires additional tedious steps and requires expenses which a user would like to avoid. The more steps that are required in PCR, the greater the likelihood of contamination from one assay to another since PCR is a highly powerful means of amplifying nucleic acids in a specimen besides the target nucleic acid. Workers in the field are expending considerable effort to reduce the problem of contaminants. Many conventional methods of PCR also use either or both a capture probe or detection probe which requires a third oligonucleotide (besides the primers) which is complementary to at least one of the amplified strands. There are many instances where it would be advisable to avoid detection or capture probes.

WO 92/16654 describes methods for amplification of target nucleic acids and detection of the resulting pyrophosphate by-product using chemiluminescent or colorimetric signal generating reagents in a liquid phase. However, the described system is undesirable because pyrophosphate is readily hydrolyzed to orthophosphate in an aqueous environment. Thus, the measurement of unstable pyrophosphate may not accurately reflect the amount of amplification which has occurred.

It would be desirable to be able to provide a more accurate measurement of the amount of target nucleic acid after PCR. Since pyrophosphate is a relatively unstable by-product, its detection is to be avoided.

The problems noted above are overcome with a method for the detection of a target nucleic acid comprising:
A) amplifying a target nucleic acid in a reaction mixture containing primers specific to and hybridizable with the opposing strands of a target nucleic acid, a dDNA polymerase and one or more dNTP's so as to generate inorganic orthophosphate, and
B) detecting the presence of inorganic orthophosphate generated in the reaction mixture amplification by contacting the inorganic orthophosphate with a reagent which provides a colorimetric signal in response to inorganic orthophosphate, as an indication of the presence of the target nucleic acid.

A diagnostic test kit useful for the detection of a target nucleic acid comprises at least one reagent needed for amplification of a target nucleic acid, the test kit characterized in that it includes, individually packaged, at least one reagent needed to provide a colorimetric signal in response to inorganic orthophosphate.

The present invention provides a means for detecting amplified target nucleic acids without expensive or difficult to handle detection reagents (such as radiolabels, capture and detection probes and detection gels) and complicated schemes. It is also possible to avoid the detection of the instable pyrophosphate and thereby provide a more precise assay. It is also possible to provide "homogeneous" assays so that the amplification products need not be separated from the other materials in the reaction system, as is typical with "heterogeneous" assay systems.

These advantages are achieved by detecting the stable inorganic orthophosphate by-product of the amplification procedure instead of detecting the amplified target nucleic acid itself or pyrophosphate by-product. The detected inorganic orthophosphate is generated in amplification from the attachment of a dNTP to a primer in the formation of a primer extension product. It can be detected using a number of conventional detection reagents in either solution or dry formats. It is particularly advantageous to detect the inorganic orthophosphate using a dry analytical element which contains reagents useful for generating a readily detectable colorimetric signal in response to the presence of inorganic orthophosphate. One such analytical element is commercially available and readily adaptable to this assay with minimal redesign. Use of the element allows the assay to be readily automated on analytical equipment.

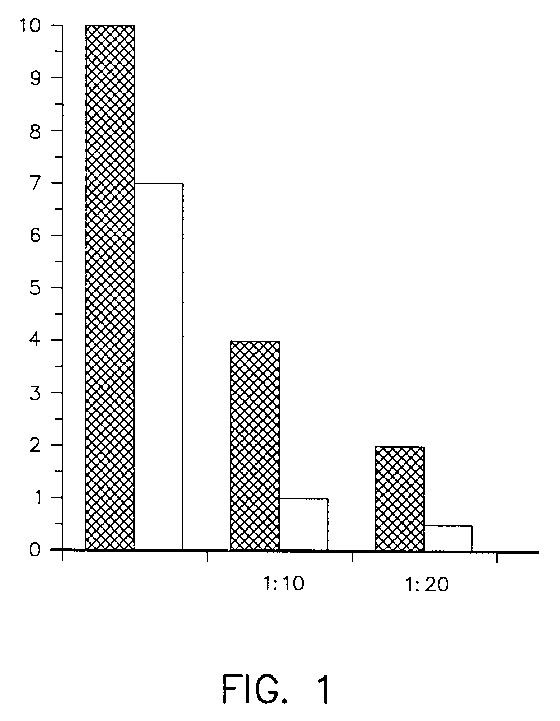

FIGURE 1 is a bar graph showing color signal intensity for several assays as described in the Example below.

The general principles and conditions for amplification and detection of nucleic acids using polymerase chain reaction are quite well known, the details of which are provided in numerous references including US-A-4,683,195, US-A-4,683,202, US-A-4,965,188, and WO-A-91/12342.

Other amplification procedures which can be used in the practice of this invention include ligase chain reaction as described, for example, in EP-A-0 320 308 and EP-A-0 439 182, and self-sustained sequence replication as described, for example, by Birkenmeyer and others, *J.Virol.Meth.* 35, pp. 117-126 (1991). While the remainder of this disclosure, however, will be directed to practicing the invention using PCR, it would be readily apparent to one skilled in the art how the teaching herein could be adapted to the other useful amplification techniques during which inorganic orthophosphate is generated.

The present invention is directed to the amplification of one or more specific nucleic acid sequences present in one or more target nucleic acids in a test specimen. Nucleic acids to be amplified and detected can be obtained from various sources including plasmids and naturally occurring DNA or RNA from any source (such as bacteria, yeast, viruses, plants and higher animals, humans). It may be extracted from various tissues including components of blond (such as peripheral blood mononuclear cells), other tissue material from other sources known in the art using known procedures. The present invention is particularly useful for the amplification and detection of nucleic acid sequences found in human genomic DNA, bacterial DNA, fungal DNA, viral RNA, or DNA or RNA found in bacterial or virus-infected cells.

Bacteria which can be detected include, but are not limited to, bacteria found in human blood, Salmonella species, Streptococcus species, Chlamydia species, Gonococcal species, *Mycobacterium tuberculosis*, *Mycobacterium avium* complex, *Legionella pneumophila*, *Clostridium difficile*, *Borreglia burgdorferei*, *Pneumocystis carinii*, *Mycoplasma Haemophilus influenzae*, Shigella species and Listeria species. Viruses which are detectable include, but are not limited to, herpes simplex viruses, Epstein Barr virus, influenza viruses, human cytomegalovirus, human papilloma viruses, hepatitis viruses, and retroviruses such as HTLV-I, HTLV-II, HIV-I and HIV-II. Protozoan parasites, yeasts and molds are also detectable.

The invention is particularly useful for the detection of the presence of DNA associated with various infectious agents, such as bacteria or viruses, with the amplification and detection of viral DNA being of most interest. Detection of DNA associated with HIV-I (and other retroviruses), human cytomegalovirus or *Mycobacterium tuberculosis* DNA is advantageously accomplished with this invention.

A "PCR reagent" refers to any of the reagents considered essential to PCR, namely one or more primers for the opposing strands of the target nucleic acid, a thermostable DNA polymerase, a DNA polymerase cofactor, and one or more deoxvribonucleoside-5'-triphosphates.

The term "primer" refers to an oligonucleotide, whether naturally occurring or synthetically produced, which is capable of acting as a point of initiation of synthesis when placed under conditions in which synthesis of a primer extension product complementary to a nucleic acid strand (that is, template) is induced. Such conditions include the presence of nucleotides (such as the four standard deoxyribonucleoside-5'-triphosphates), a thermostable DNA polymerase, a DNA polymerase cofactor, and suitable temperature and pH. Generally, the primers used in this invention will have from 12 to 60 nucleotides.

The DNA polymerase is preferably "thermostable", meaning that it is generally stable to heat and preferentially active at higher temperatures, especially the high temperatures used for denaturation of DNA strands. More particularly, the thermostable DNA polymerases are not substantially inactivated at the high temperatures used in polymerase chain reactions as described herein.

A number of thermostable DNA polymerases have been reported in the art, including those mentioned in detail in US-A-4,965,188 and US-A-4,889,818. Particularly useful polymerases are those obtained from various Thermus bacterial species, such as *Thermus aquaticus*, *Thermus thermophilus*, *Thermus filiformis* or *Thermus flavus*. Other useful thermostable polymerases are obtained from a variety of other microbial sources including *Thermococcus literalis*, *Purococcus furiosus*, Thermotoga sp. and those described in WO-A-89/06691.

A DNA polymerase cofactor refers to a nonprotein compound on which the enzyme depends for activity. A number of such materials are known cofactors including manganese and magnesium compounds. Such compounds contain the manganese or magnesium in such a form that divalent cations are released into an aqueous solution. Useful cofactors include manganese and magnesium salts, such as chlorides, sulfates, acetates and fatty acid salts (for example, butyric, caproic, caprylic, capric and lauric acid salts).

Also needed for PCR is one or more deoxyribonucleoside-5'-triphosphates, such as dATP, dCTP, dGTP, dTTP or dUTP. Analogues such as dITP and 7-deaza-dGTP are also useful. Preferably, two or more, and more preferably, at least four, deoxyribonucleoside-5'-triphosphates are used. It is conventional to identify the nucleoside triphosphates, for example dATP, dCTP, dGTP and dTTP, collectively as dNTP's.

The thermostable DNA polymerase described above is preferably used in the practice of this invention in combination with a water-soluble temperature sensitive inhibitor. This inhibitor acts to bind to and inactivate the polymerase at temperature T₁ which is generally below 85°C. For most practical purposes, T₁ is below 55°C. Therefore, the presence of background orthophosphate is kept to a minimum.

Advantageously, however, the water-soluble temperature sensitive inhibitor dissociates from the DNA polymerase and becomes ineffective to inactivate the DNA polymerase at temperature T₂ which is generally above 40°C. Preferably, T₂ is at least 5°C above T₁. A representative inhibitor is shown in the example below, in which T₁ is generally from 40°C to 55°C and T₂ is generally from 75 to 95°C.

The inhibitor can be any biological or chemical molecule which will complex with the thermostable DNA polymerase to effect the noted temperature-dependent responses in the polymerase. The inhibitor can be DNA polymerase-binding proteins which bind and release the DNA polymerase in response to temperature. Particularly useful inhibitors are antibodies specific to the DNA polymerase which have the noted binding and releasing properties.

Two such monoclonal antibodies are readily obtained by a skilled artisan using conventional procedures and starting materials including either of hybridoma cell lines HB 11126 and 11127 deposited with the American Type Culture Collection (Rockville, Maryland). The monoclonal antibody is generally present in an amount of from about 5:1 to about 500:1 molar ratio to the DNA polymerase (preferably, from 25:1 to 100:1 molar ratio).

The PCR reagents described herein are provided and used in PCR in any concentration suitable for a given process. The minimal amounts of primers, cofactors and deoxyribonucleotide-5'-triphosphates needed for amplification and suitable ranges of each are well known in the art. Preferably, from 0.1 to 50 units of polymerase per 100 µl of reaction mixture are needed for PCR, depending upon the particular activity of a given enzyme. A "unit" is defined herein as the amount of enzyme activity required to incorporate 10 nmoles of total nucleotides (dNTP's) into an extending nucleic acid chain in 30 minutes at 74°C. More preferably, from 10 to 25 units of DNA polymerase/100 µl of solution are used. The amount of each primer is at least 0.075 µmolar with from 0.1 to 2 µmolar being preferred, but other amounts are well known in the art. The DNA polymerase cofactor is generally present in an amount of from 2 to 15 mmolar, and each dNTP is generally present at from 0.25 to 3.5 mmolar (from 1 to 14 mmolar for the total of the four common dNTP's).

The PCR reagents can be supplied individually, or in a buffered solution having a pH in the range of from 7 to 9.

A target nucleic acid (that is, one to be amplified) can be obtained from any of a variety of sources as noted above. Generally, it is extracted in some manner to make it available for contact with the primers and other PCR reagents. This usually means removing unwanted proteins and cellular matter from the specimen in a suitable manner. Various procedures are known in the art, including those described by Laure and others in *The Lancet*, pp. 538-540 (Sept. 3, 1988), Maniatis and others, Molecular Cloning: A Laboratory Manual, pp. 280-281 (1982), Gross-Belland and others in *Eur.J.Biochem.,* 36, 32 (1973) and US-A-4,965,188. Extraction of DNA from whole blood or components thereof are described, for example, in EP-A-0 393 744, Bell and others, *Proc. Natl. Acad. Sci. USA,* 78(9), pp. 5759-5763 (1981) and Saiki and others, *Bio/Technology*, 3, pp. 1008-1012 (1985).

Since the nucleic acid to be amplified or detected is usually in double stranded form, the two strands must be separated (that is, denatured) before priming can take place. Denaturation is accomplished using a heat treatment alone or in combination with any suitable other physical, chemical or enzymatic means as described in the art. Initial denaturation is generally carried out by heating the specimen suspected of containing the targeted nucleic acid at a first temperature of from 85 to 100°C for a suitable time, for example from 1 second to 3 minutes.

The denatured strands are then cooled to a second temperature which is generally in the range of from 55 to 70°C. Once cooled, the reaction mixture containing PCR reagents is incubated at a suitable temperature to effect formation of primer extension products. Generally, this temperature is at least 50°C, and preferably in the range of from 65 to 75°C. The time for incubation can vary widely depending upon the incubation temperature and the length of extension products desired, but in preferred embodiments, it is from 1 to 120 seconds. Each cycle of PCR can be carried out using either two or more different temperatures, one temperature for denaturation, and a second and an additional temperature for priming or primer extension product formation. That is, some PCR processes utilize a second temperature for priming and a third temperature for primer extension. Preferably, in the practice of this invention, the same temperature is used for both priming and primer extension.

If the hybridized primer extension products are denatured, PCR can be carried out further in as many cycles of priming, extension and denaturation as desired. Generally, at least 20 cycles will be carried out, with from 20 to 50 cycles being preferred.

At any time during the assay, but after at least one amplification cycle, generated inorganic orthophosphate can be detected as described below.

The amplification method of this invention is preferably conducted in a continuous, automated manner so that the reaction mixture is temperature cycled in a controlled manner for desired preset times. A number of instruments have been developed for this purpose, as one of ordinary skill in the art would know.

One such instrument for this purpose is described in some detail in US-A-4,965,188 and EP-A-0 236 069. Generally, this instrument includes a heat conducting container for holding a number of reaction tubes containing reaction mixture, a means for heating, cooling and temperature maintenance, and a computing means to generate signals to control the amplification sequence, changes in temperature and timing.

A preferred instrument for processing amplification reactions in a disposable chemical test pack is described in some detail in US-A-5,089,233. In general, this instrument comprises a surface for supporting chemical test packs, pressure applicators supported above the surface for acting on the reaction pack to transfer fluids between adjacent chambers in the test pack, and means for operating the pressure applicators through a range of movement extending across the test pack.

EP-A-0 402 994 provides details of useful chemical test packs which can be processed using the instrument described in US-A-5,089,233. Also described therein are means for heating and cooling the test pack at repeated intervals (that is, through cycles) appropriate for the method of the present invention.

It is also useful for the method of this invention to be carried out in a suitable container. The most crude container would be a test tube, cuvette, flask or beaker, but more sophisticated containers have been fashioned in order to facilitate automated procedures for performing the method (see for example, WO-A-91/12342). For example, cuvette and chemical test packs (also known as pouches), constructed to provide certain temperature characteristics during the practice of the method, are described in US-A-4,902,624, US-A-5,173,260 and US-A-5,229,297. Such test packs have a multiplicity of reaction chambers having various reagents, buffers and other materials which are useful at various stages in the amplification or detection method. The packs can be appropriately and rapidly heated and cooled in cycles to promote the various steps of the amplification method of this invention. Other useful containers could be suitably fashioned for automated or single use of the method of this invention.

The amplification portion of the assay could also be carried out in test tubes and detection in a different device such as disposable test devices such as that commercially available under the mark SURECELL™ (Eastman Kodak Company) or any other device (for example, microwells).

Inorganic orthophosphate generated from amplification by polymerase chain reaction can be detected using any of a number of conventional methods and reagents known for phosphate detection. In its simplest embodiment, the invention can be practiced in a solution format by detecting the orthophosphate using reagents which provide a colorimetric signal (that is, signal in the range of 200 to 700 nm wavelength in the electromagnetic spectrum) in response to one or more reactions with orthophosphate. One such method is described in more detail, for example by Horder, *Anal*. *Biochem*. 49, pp. 37-47 (1972) and involves the reaction of the orthophosphate with ammonium molybdate to produce an ammonium phosphomolybdate complex, followed by reduction of the complex with ascorbic acid and sodium arsenite-sodium citrate to the blue molybdate complex which is evaluated at 700 nm.

Another method is described by Young in *J.Clin.Path*. 19, 397 (1966) and involves the complexation of orthophosphate with a water-soluble molybdate ion at an acidic pH to give a yellow complex which can be subsequently reduced by a variety of reducing agents to a complex having a blue color. The absorbance of the reduced complex can be measured using suitable equipment and the density of the color is proportional to the amount of orthophosphate in the sample. Details of the use of molybdate salts are provided, for example, in US-A-3,853,469 and US-A-3,953,359.

Such solution methods are typically carried out in a suitable container (for example, test tube or cuvette) by mixing the reagents needed to detect inorganic orthophosphate with a sample of the amplification reaction mixture. This is typically done at room temperature for a sufficient time to obtain the desired colorimetric signal which is measured using conventional equipment.

Useful water-soluble molybdate salts include, but are not limited to, aluminum molybdate, cadmium molybdate, calcium molybdate, barium molybdate, bismuth molybdate, lithium molybdate, potassium molybdate, sodium molybdate, zinc molybdate, alkylammonium molybdate (with the alkyl having from 1 to 6 carbon atoms), and others readily apparent to one skilled in the art.

Representative reducing agents in the practice of this invention which reduce the complex of molybdate and inorganic orthophosphate include, but are not limited to, p-methylaminophenol sulfate, stannous chloride, phenylhydrazine, hydroquinone, ferrous sulfate, ascorbic acid, aminonaphtholsulfonic acid, 2,4-diaminophenol, N-phenyl-p-phenylenediamine and other known phenolic and phenylenediamine compounds which are known in the photographic art as silver halide developers, and especially the acid addition salts of the amine-containing developers.

In a preferred embodiment, inorganic orthophosphate is detected by applying a sample of the amplification reaction mixture to a dry element wherein the reactions needed to detect orthophosphate are conducted. The reagents necessary for the reactions can be added to the element separate from the sample, or mixed with the sample prior to application to the element. More preferably, the element contains the necessary reagents for the reactions. Such elements can be single layer filter papers impregnated with the necessary reagents, or they can be more complicated multilayer analytical elements, such as those commercially available as EKTACHEM™ Clinical Chemistry slides for Phosphorus (available from Eastman Kodak Company).

Most preferably, the dry element useful in the practice of this invention has an inert support with at least one reagent layer and a porous spreading layer disposed thereon. The support is generally dimensionally stable, inert to chemical reaction and preferably transparent. However, non-transparent supports can be used if the mode of detection is reflectance spectroscopy instead of transmission spectroscopy.

The porous spreading zone is prepared from any of the known materials used for such zones as described, for example in US-A-4,292,272, US-A-3,992,158, US-A-4,258,001, US-A-4,430,436, and JP 57(1982)-101760. Preferred spreading zones are those described in US-A-3,992,158 as "blush polymer" zones.

The elements have at least one other layer which can contain one or more reagents needed for the assay. Preferably, there are at least two reagent layers. All of the layers are generally in fluid contact with each other, meaning that fluids, reagents and reagent products can pass or be transported between superposed regions of adjacent layers, unless of course, a reagent is immobilized in some manner so it will not migrate within or without a layer.

A variety of different elements, depending upon the method and equipment for assay, can be prepared in accordance with this invention. They can be configured in a variety of forms and shapes, including elongated tapes of any desired width, sheets, slides or chips. Preferred elements are configured as test slides like those commercially available under the EKTACHEM™ trademark for a variety of clinical assays. Such test slides are described in a considerable number of patents and other publications.

In a most preferred embodiment, a multilayer analytical element useful in the practice of this invention comprises a nonporous, transparent support having thereon, in order and in fluid contact:
a first reagent layer containing a water-soluble molybdate salt mixed in a hydrophilic binder (many useful binders are known in the art),
a second reagent layer containing a reducing agent mixed in a hydrophilic binder, and
a porous spreading layer.

The assay of this invention using the analytical element can be manual or automated. In general, the element is used by physically contacting it with a sample of the PCR reaction mixture containing inorganic orthophosphate under ambient conditions (although other temperatures can be used). The specimen and reagents become mixed within the layers of the element and any inorganic orthophosphate present in the specimen reacts with the water-soluble molybdate salt to produce a complex which is then reduced by the reducing agent. Contact can be achieved in any suitable manner, for example by dipping or immersing the element into the specimen or preferably, by spotting the specimen onto the element by hand, machine or suitable dispensing means.

Generally within 10 minutes (preferably 5 minutes), a spectrophotometric measurement is made. This measurement can be made using suitable reflection or transmission spectrophotometric equipment and procedures as a measure of inorganic orthophosphate concentration in the sample which is then a direct measure of the target nucleic acid. Generally, the detectable signal is measured at a wavelength in the range of from 650 to 700 nm, with a measurement at 680 nm being preferred.

In the following examples, all percentages are by weight unless otherwise noted.

### Materials and Methods for Examples:

Recombinant DNA polymerase from *Thermus aquaticus* was prepared using known procedures, such as those described in EP-A-0 482 714 and had an activity of 250,000 units/mg of protein.

The primers used in the Example below were prepared using known starting materials and procedures using an Applied Biosystems Model 380B, three column DNA synthesizer using standard phosphoramidite chemistry. Nucleoside-3'-phosphoramidites and nucleoside derivatized controlled pore glass supports were obtained from Applied Biosystems. The primers prepared in this manner were functionalized at the 5' end with two tetraethylene glycol spacers following by a single commercially available DuPont biotin phosphoramidite. All purifications were carried out using a nucleic acid purification column, followed by reversed phase HPLC techniques. The primers had the following sequences:
SEQ ID NO:1:
5'-AGTGGGGGGA CATCAAGCAG CCATGCAA-3'
SEQ ID NO:2:
5'-TTCCTGCTAT GTCACTTCCC CTTGGTTC-3'.

A target nucleic acid (HIV-I DNA) was isolated from HIV-I cell line HUT-AAV 78 lymphocytes using conventional procedures. The primers were complementary to opposing strands of the target nucleic acid along nucleic acid sequences in the gag region.

A monoclonal antibody specific to the noted DNA polymerase was prepared using conventional procedures, such as those described by Milstein and others, *Nature*, 256, pp. 496-497, 1975, and hybridoma cell lines (either HB 11126 and 11127 from ATCC), so that antibody secreting cells of the host animal were isolated from lymphoid tissue (such as the spleen) and fused with SP2/0-Ag14 murine myeoloma cells in the presence of polyethylene glycol, diluted into selective media and plated in multiwell tissue culture dishes. About 7-14 days later, the hybridoma cells containing the antibodies were harvested, and purified using conventional techniques.

### Example

In this example, the target nucleic acid, HIV-I DNA noted above, was amplified using polymerase chain reaction in the following manner. The target nucleic acid (about 10,000 copies) was mixed into the following PCR reaction mixture.

The PCR reagent mixture contained tris(hydroxy-methyl)aminomethane hydrochloride buffer (10 mmolar, pH 8), tris(hydroxymethyl)aminomethane buffer (6.86 mmolar), potassium chloride (50 mmolar), magnesium chloride (10 mmolar), gelatin (100 µg/ml), dATP, dCTP, dGTP and dTTP (1.5 mmolar of each), ethylenediaminetetraacetic acid (686 µmolar), glycerol (9.5%), the primers identified above (0.4 µmolar of each), DNA polymerase identified above (48 units/300 µl), and a monoclonal antibody specific to DNA polymerase identified above (50:1 molar ratio to DNA polymerase).

The amplification and detection procedure for the assay were as follows:

### Amplification:

Denature by heating at 95°C for 60 seconds, 40 cycles of priming and extending at 68°C for 30 seconds, and heating at 94°C for 15 seconds using a commercially available PCR processor.

### Detection:

A sample (10 µl) of the resulting reaction mixture containing amplified target nucleic acid was applied to a commercially available EKTACHEM™ Clinical Chemistry Slide for Phosphate. The element is described below in more detail.

After 5 minutes of incubation at about 55°C, the dye signal generated by the presence of inorganic orthophosphate in the sample was observed by the unaided eye.

Additional samples of the PCR reaction mixture were diluted 1:10 and 1:20 and similarly tested. Also tested was a sample of undiluted buffer containing no PCR reagents and undiluted and diluted (1:10 and 1:20) PCR reaction mixture without target nucleic acid. The results are shown in FIG. 1 with color signal intensity on the "y" axis. The first set of bars were for undiluted samples.

The dark bars in FIG. 1 show the color signal generated from the presence of HIV-I DNA in the test samples. The light bars show background for the diluted and undiluted PCR reagent mixture. No signal was obtained for the buffer alone.

| Element for Orthophosphate Detection | | Dry Coverage (g/m²) |
|---|---|---|
| Spreading Layer | Barium sulfate | 109 |
| | Cellulose acetate | 8.6 |
| | ESTANE™ 5715 polyurethane | 1.1 |
| | TRITON™ X-405 nonionic surfactant | 2.2 |
| Second Reagent Layer | Poly(vinyl pyrrolidone) | 4.4 |
| | p-Methylaminophenol sulfate | 3.5 |
| | 5,5'-Dimethyl-1,3-cyclohexanedione | 1.5 |
| | TRITON ™ X-100 nonionic surfactant | 0.23 |
| First Reagent Layer | Binder* | 9 |
| | Glutaric acid | 3.4 |
| | TRITON ™ X-100 nonionic surfactant | 0.22 |
| | Ammonium molybdate | 3.36 |
| | Sodium hydroxide | 0.32 |
| Poly(ethylene terephthalate) Support | | |

| | | |
|---|---|---|
| * Poly[acrylamide-co-N-vinyl-2-pyrrolidone] (50:50 weight ratio) | | |

To demonstrate that the noted analytical element detects orthophosphate instead of pyrophosphate, 1 drop of a solution of sodium pyrophosphate (0.01 molar) was applied to the EKTACHEM PHOS™ analytical element described above. The resulting dye density was determined after 1 minute at 660 nm using a digital refractometer to be 0.076 (absorbance units).

One drop of a solution of sodium orthophosphate (0.01 molar) was applied to another EKTACHEM PHOS™ analytical element, and the resulting dye density was determined to be 1.02 using the same procedure.

## Claims

1. A method for the detection of a target nucleic acid comprising:
A) amplifying a target nucleic acid in a reaction mixture containing primers specific to and hybridizable with the opposing strands of a target nucleic acid, a DNA polymerase and one or more dNTP's so as to generate inorganic orthophosphate, and
B) detecting the presence of inorganic orthophosphate generated in said reaction mixture from the amplification by contacting the inorganic orthophosphate with a reagent which provides a colorimetric signal in response to inorganic orthophosphate, as an indication of the presence of the target nucleic acid.

2. The method as claimed in claim 1 wherein the target nucleic acid is amplified using polymerase chain reaction in the presence of a thermostable DNA polymerase.

3. The method as claimed in either of claims 1 and 2 wherein the inorganic orthophosphate is detected by its complexation with a water-soluble molybdate salt and subsequent reduction of the resulting complex.

4. The method as claimed in claim 3 wherein the inorganic orthophosphate is detected by contacting the reaction mixture with an analytical element containing the water-soluble molybdate salt.

5. The method as claimed in claim 4 wherein the analytical element used for inorganic orthophosphate detection comprises:
a first reagent layer comprising the water-soluble molybdate salt admixed with a hydrophilic binder,
a second reagent layer comprising a reducing agent for the complex formed from the inorganic orthophosphate and the water-soluble molybdate salt, the reducing agent admixed with a hydrophilic binder, and
a porous spreading layer.

6. The method as claimed in any of claims 3 to 5 wherein the water-soluble molybdate salt is ammonium molybdate and the reducing agent is p-methylaminophenol sulfate.

7. The method as claimed in claim 1 wherein the target nucleic acid is amplified using ligase chain. reaction.

8. A diagnostic test kit useful for the detection of a target nucleic acid comprising at least one PCR reagent needed for amplification of a target nucleic acid, the test kit characterized in that it includes, individually packaged, at least one reagent needed to provide a colorimetric signal in response to inorganic orthophosphate.

9. The kit as claimed in claim 8 wherein the reagent for providing a colorimetric signal is a water-soluble molybdate salt or a reducing agent for the complex formed from the inorganic orthophosphate and the molybdate salt.

10. The kit as claimed in either of claims 8 and 9 comprising a dry analytical element containing the colorimetric signal providing reagent.
